(19) Europäisches Patentamt
European Patent Office
Office européen des brevets

(11) EP 1 324 980 B1

(12) **EUROPEAN PATENT SPECIFICATION**

(45) Date of publication and mention of the grant of the patent:
**06.02.2008 Bulletin 2008/06**

(21) Application number: **01986297.8**

(22) Date of filing: **27.09.2001**

(51) Int Cl.:
***C07C 275/16*** *(2006.01)* ***C12P 13/00*** *(2006.01)*
***C12P 13/10*** *(2006.01)* ***C12N 1/20*** *(2006.01)*
***A61K 31/17*** *(2006.01)* ***A61K 31/197*** *(2006.01)*

(86) International application number:
**PCT/EP2001/011192**

(87) International publication number:
**WO 2002/028824 (11.04.2002 Gazette 2002/15)**

(54) **CITRULLIMYCINES, A PROCESS FOR THEIR PRODUCTION AND THEIR USE AS PHARMACEUTICALS**

CITRULLIMYCINE, VERFAHREN ZU IHRER HERSTELLUNG UND IHRE VERWENDUNG ALS ARZNEIMITTEL

CITRULLIMYCINES, LEUR PROCEDE DE PRODUCTION ET LEUR UTILISATION EN TANT QU'AGENTS PHARMACEUTIQUES

(84) Designated Contracting States:
**AT BE CH CY DE DK ES FI FR GB GR IE IT LI LU MC NL PT SE TR**

(30) Priority: **02.10.2000 EP 00121566**

(43) Date of publication of application:
**09.07.2003 Bulletin 2003/28**

(73) Proprietor: **Sanofi-Aventis Deutschland GmbH**
**65929 Frankfurt am Main (DE)**

(72) Inventors:
- **HOPMANN, Cordula**
**D-10629 Berlin (DE)**
- **KURZ, Michael**
**D-65719 Hofheim (DE)**
- **BRÖNSTRUP, Mark**
**D-60316 Frankfurt (DE)**
- **WINK, Joachim**
**D-63322 Rödermark (DE)**

(56) References cited:
- **No relevant documents disclosed**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to novel active compounds (Citrullimycines) which are obtainable by cultivation of Streptomycetes sp., DSM 13309, and to their pharmaceutically acceptable salts and derivatives. The present invention further relates to a process for the production of the Citrullimycines, to the Streptomycetes sp., DSM 13309, to the use of the Citrullimycines as a pharmaceutical, in particular to their use as substances with an affinity for neurotensin receptors, and to Citrullimycine-containing pharmaceuticals.

Neurotensin is a brain and gastrointestinal 13-amino acid hormonal peptide, which is involved in the control of a broad variety of physiological activities as a central neurotransmitter or neuromodulator in both the central nervous system and in the periphery. It fulfills many functions through their interaction with specific receptors which have been characterized in several tissues and cell lines of peripheral and central organs. Studies with neurotensin have led to implications of their involvement in schizophrenia, Parkinson and Alzheimer diseases. Compounds with an affinity for the neurotensin receptors might therefore be useful as therapeutics of these diseases.

It has now been found that the microorganism strain Streptomycetes sp., DSM 13309, is able to form novel active substances which inhibit the human neurotensin receptor proteins expressed in Human Embryonic Kidney (HEK) cell membranes.

The present invention accordingly relates to the active substances (Citrullimycines) formed by the strain Streptomycetes sp., DSM 13309, and to their physiologically tolerated salts, esters, ethers and other obvious chemical equivalents.

The present invention accordingly relates to compounds of the formula I

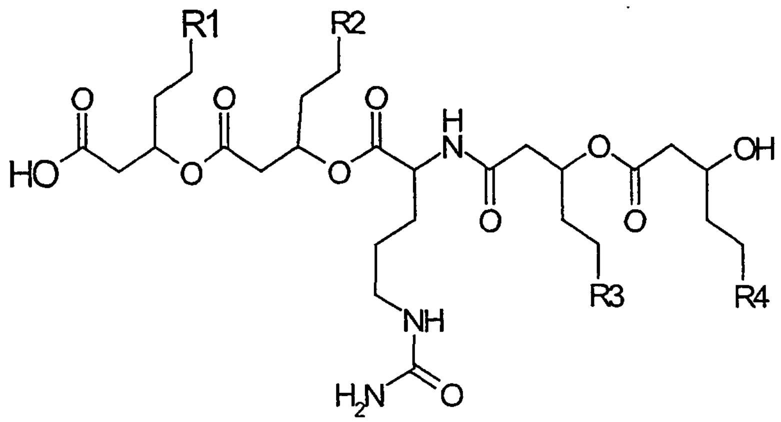

where

$R_1$, $R_2$, $R_3$ and $R_4$ are, independently of one another, alkyl residues with 1 to 6, preferably 2 to 5, carbon atoms; and the physiologically tolerated salts thereof and derivatives such as esters, ethers and other obvious chemical equivalents, including all stereoisomeric forms and all tautomeric forms.

The alkyl residues in the compounds of the formula I can be straight-chain or branched.

Examples of alkyl residues are: methyl, ethyl, propyl, butyl, pentyl, hexyl, isopropyl, isobutyl, isopentyl, sec-butyl, tertbutyl and neopentyl.

The invention further relates to

1) a compound of the formula I above in which one of the residues $R_1$, $R_2$, $R_3$ or $R_4$ is a straight or branched propyl residue and the rest of the residues are straight or branched butyl residues ( =Citrullimycine A: molecular formula: $C_{41}H_{75}N_3O_{11}$, MW: 785) and the physiologically tolerated salts and derivatives thereof;

2) a compound of the formula I above in which either:

(a) two of the residues $R_1$, $R_2$, $R_3$ or $R_4$ are straight or branched butyl residues and two of the residues are straight or branched propyl residues; or
(b) $R_1$, $R_2$, $R_3$ and $R_4$ are one butyl, one pentyl, one ethyl and one propyl residue, in any order, the residues being either straight chain or branched;

(= Citrullimycine B: molecular formula: $C_{40}H_{73}N_3O_{11}$, MW 771) and the physiologically tolerated salts and derivatives thereof; and

3) a compound of the formula I above in which either:

(a) $R_1$, $R_2$, $R_3$ and $R_4$ are straight or branched butyl residues; or
(b) two of the residues $R_1$, $R_2$, $R_3$ and $R_4$ are straight or branched butyl residues, one is a straight or branched propyl residue and one is a straight or branched pentyl residue;

(= Citrullimycine C: molecular formula: $C_{42}H_{77}N_3O_{11}$, MW 799) and the physiologically tolerated salts and derivatives thereof.

It is, of course, understood that the compounds of formula I may exist in a variety of isomeric configurations, including structural as well as stereo-isomers. It is further understood that the present invention encompasses those compounds of formula I in each of their various structural and stereo isomeric configurations as individual isomers and as mixtures of isomers.

The Citrullimycines A to C according to the present invention are generally isolated as a mixture of isomers. In respect of Citrullimycine A, for example, an isolated sample may comprise a mixture of two or more of the following isomers:

Isomer 1: $R_1$, $R_2$ and $R_4$ = $-CH(CH_3)CH_2CH_3$, $R_3$ = $-CH(CH_3)CH_3$
Isomer 2: $R_1$, $R_2$ and $R_3$ = $-CH(CH_3)CH_2CH_3$, $R_4$ = $-CH(CH_3)CH_3$
Isomer 3: $R_1$, $R_3$ and $R_4$ = $-CH(CH_3)CH_2CH_3$ , $R_2$ = $-CH(CH_3)CH_3$
Isomer 4: $R_1$ = $-CH(CH_3)CH_3$ , $R_2$, $R_3$ and $R_4$ = $-CH(CH_3)CH_2CH_3$

The isomers of Citrullimycine A may exist in any ratio in the mixture isolated.

Citrullimycines A-C may be characterized by any one or more of their physico-chemical and spectral properties, such as their mass spectrometry or $^1H$ NMR and $^{13}C$ NMR spectroscopic data (see Tables 1 and 2 below).

The compounds of the formula I may be described as a sequence of four β-hydroxyacids with a citrulline molecule incorporated in the middle of the sequence. Citrullimycines A-C differ in the length of the alkylchain of the β-hydroxyacids. The compounds of the formula I are obtainable by cultivation of the microorganism Streptomycetes sp.. The said microorganism has been deposited on the 14 February 2000 with the German Collection of Microorganisms and Cell Cultures (DSMZ - Deutsche Sammlung von Mikroorganismen und Zellkulturen GmbH), Braunschweig, Germany and has been given the accession number DSM 13309.

Thus, the present invention further provides a process for the production of said compounds of the formula I, which comprises cultivating the microorganism Streptomycetes species DSM 13309, its mutants and variants, under aerobic conditions in a nutrient medium containing one or more sources of carbon and one or more sources of nitrogen and, optionally, nutrient inorganic salts and/or trace elements, and then isolating and purifying the said compounds in a customary manner.

The nutrient medium preferably contains sources of carbon, nitrogen and nutrient inorganic salts. The carbon sources are, for example, starch, glucose, sucrose, dextrin, fructose, molasses, glycerol, lactose or galactose, preferably glucose. The sources of nitrogen are, for example, soyabean meal, peanut meal, yeast extract, beef extract, peptone, malt extract, corn steep liquor, gelatin or casamion acids, preferably soyabean meal. The nutrient inorganic salts are, for example sodium hydrogen phosphate, potassium hydrogen phosphate, ammonium hydrogen phosphate, sodium chloride, calcium chloride, calcium carbonate, potassium nitrate, ammonium sulphate or magnesium sulphate, preferably calcium carbonate and cobalt(II)chloride.

The cultivation of strain DSM 13309 may be carried out at temperatures between 20-35°C and pH between 5.0 and 8.0. Preferably strain DSM 13309 is cultivated at 27°C (±1°C) and pH 6.8 -7.0.

The cultivation of strain DSM 13309 is preferably carried out for 48-240 hours when an optimal yield of the Citrullimycines of the invention is obtained. It is particularly preferred to carry out the cultivation by fermentation for 60-120 hours under submerged conditions for example in shake flasks as well as in laboratory fermenters. The progress of fermentation and formation of the Citrullimycines can be detected by High Pressure Liquid Chromatography (HPLC) or LC-MS and by measuring the bioactivity of the culture broth. In the resulting culture broth The Citrullimycines are present in the culture filtrate as well as in the mycelium. They can be isolated using known separation techniques. Thus, they can be recovered from the culture filtrate by extraction with a water immiscible solvent such as ethyl acetate, dichloromethane, chloroform or butanol at pH 3-8 or by hydrophobic interaction chromatography using polymeric resins such as "Diaion HP-20®" or "MCI® Gel CHP-20P" (Mtheirubishi Chemical Industries Limited, Japan), "Amberlite XAD®" (Rohm and Hass Industries U.S.A.), activated charcoal or ion exchange chromatography at pH 3-8. The preferred method

is chromatography on MCl® Gel CHP-20P. The active material can also be recovered from mycelium by extraction with a water miscible solvent such as methanol, acetone, acetonitrile, n-propanol or iso-propanol or a water immiscible solvent such as ethyl acetate, dichloromethane, chloroform or butanol at pH 3-8 and the preferred method is the extraction with methanol. Concentration and lyophilization of the extracts gives the active crude material.

The Citrullimycines of the present invention may, for example, be recovered from the crude material as follows:

By fractionation using any of the following techniques: normal phase chromatography (using alumina or silica gel as stationary phase and eluents such as petroleum ether, ethyl acetate, methylene chloride, acetone, chloroform, methanol or combinations thereof and additions of amines such as $NEt_3$), reverse phase chromatography (using reverse phase silica gel like dimethyloctadecylsilylsilica gel, also called RP-18 or dimethyloctylsilyl silica gel also called RP-8 as stationary phase and eluents such as water, buffers viz. phosphate, acetate, citrate (pH 2-8) and organic solvents such as methanol, acetonitrile, acetone, tetrahydrofuran or combinations of these solvents), gel permeation chromatography using resins such as ®Sephadex LH-20 (Pharmacia Chemical Industries, Sweden), TSKgel ®Toyopearl HW (TosoHaas, Tosoh Corporation, Japan) in solvents such as methanol, chloroform, acetone, ethyl acetate or their combinations or ®Sephadex G-10 and G-25 in water; or by counter-current chromatography using a biphasic eluent system made up of two or more solvents such as water, methanol, ethanol, iso-propanol, n-propanol, tetrahydrofuran, acetone, acetonitrile, methylene chloride, chloroform, ethyl acetate, petroleum ether, benzene and toluene. These techniques may be used repeatedly or a combination of the different techniques may be used. The preferred method is chromatography over reverse phase silica gel (RP-18).

Obvious chemical equivalents ('derivatives') of the compounds according to the invention are compounds which show slight chemical difference, that is to say have the same effect or are converted under mild conditions into the compounds according to the invention. Said equivalents include, for example, esters, ethers, complexes or adducts of the or with the compounds according to the invention.

The compounds according to the present invention may be converted into pharmaceutically acceptable salts and derivatives, which are all covered by the present invention. The salts and derivatives can be prepared by standard procedures known to one skilled in the art.

Physiologically tolerated salts of the compounds of the formula I mean both the organic and the inorganic salts thereof as described in Remington's Pharmaceutical Sciences ($17^{th}$ edition, page 1418 (1985)). Salts like sodium and potassium salts, for example, may be prepared by treating the compounds according to the invention with suitable sodium or potassium bases.

Esters of the free carboxylic acid may be prepared by methods given in the literature for example by treatment with an alcohol in the presence of a dehydrating agent like decyclohexylcarbodiimide (DCC) as described in Advanced Organic Synthesis, 4th Edition, J. March, John Wiley & Sons., 1992, page 395.

The ester groups of the compounds according to the present invention may be reduced to ether groups as given in the literature, for example, by treatment with $BF_3$-etherat, $LiAlH_4$ $LiBH_4$ or $NaBH_4$ as described in Advanced Organic Synthesis, 4th Edition, J. March, Wiley & Sons., 1992, page 1213. The amide group may be reduced in the same way by reaction with $LiAlH_4$.

The Citrullimycines according to the invention show inhibition in the neurotensin receptor binding assay of the human neurotensin receptor proteins expressed in the HEK cell membranes. In this assay, Citrullimycine A showed an $IC_{50}$ of 16 $\mu$M.

The compounds according to the present invention and their pharmaceutically acceptable salts and derivatives can be administered to animals, preferably to mammals, and in particular to humans as pharmaceuticals on their own, in mixtures with one another and in the form of pharmaceutical compositions which permit parenteral administration. Accordingly the present invention also relates to compounds of the formula I above and their pharmaceutically acceptable salts and derivatives for use as pharmaceuticals, in particular for use as a neurotensin antagonist with an affinity for the neurotensin receptor. The present invention further relates to pharmaceutical compositions which contain an effective amount of one or more of the target compounds and/or one or more pharmaceutically acceptable salts and/or derivatives thereof, together with a pharmaceutically acceptable carrier.

The compounds according to the invention can be administered orally, intramuscularly, intravenously or by other modes of administration. Pharmaceutical compositions which contain these compounds or a pharmaceutically acceptable salt or derivative thereof, optionally with other pharmaceutically active substances, can be prepared by mixing the active compounds with one or more pharmacologically tolerated auxiliaries and/or excipients. The mixture can then be converted into a suitable pharmaceutical form such as tablets, coated tablets, capsules, granules, powders, emulsions, suspensions or solutions.

Examples of auxiliaries and/or excipients which may be mentioned are fillers, emulsifiers, lubricants, masking flavours, colorants and buffer substances tragacanth, lactose, talc, agar-agar, polyglycols, ethanol and water. Suitable and preferred for parenteral administration are suspensions or solutions in water. It is also possible to administer the active substances as such, without vehicles or diluents, in a suitable form, for example, in capsules.

The invention also relates to a method for the production of a pharmaceutical, which comprises converting at

least one of the compounds according to the invention with a pharmaceutically suitable and physiologically tolerated carrier and, where appropriate, further suitable active substances, additives or excipients into a suitable dosage form.

As is customary, the galenic formulation and the method of administration as well as the dosage range which are suitable in a specific case depend on the species to be treated and on the state of the respective condition or disease, and can be optimized using methods known in the art.

The following are illustrative examples of the present invention but not limitative of the scope thereof :

EXAMPLE 1

Maintenance of the producer strain Streptomycetes species, DSM 13309

Composition of maintenance medium

The producer strain DSM 13309 was maintained on the following medium :

| | |
|---|---|
| Malt extract : | 10.0 g |
| Glucose : | 4.0 g |
| Yeast extract : | 4.0 g |
| Agar powder : | 15.0 g |
| Demineralised water : | 1 litre |
| pH : | 7.0-7.5 |

After dissolving the ingredients thoroughly by heating, the resultant solution was distributed in test tubes and sterilized at 121°C for 20 mins. The test tubes were cooled and allowed to solidify in a slanting position. The agar slants were streaked with the growth of Streptomycetes sp., DSM 13309, by a wire loop and incubated at 28°C (±1°C) until a good growth was observed. The well grown cultures were stored in the refrigerator at +8°C.

Preparation of glycerol working seed

| Composition of medium | |
|---|---|
| Yeast extract | 4 g |
| Soluble starch | 15 g |
| $K_2HPO_4$ | 1 g |
| $MgSO_4$ x 7 $H_2O$ | 0.5 g |
| Demineralised water | 1 litre |
| PH | 7.0 |

The above medium was distributed in 100 ml amounts in 300 ml Erlenmeyer flasks and autoclaved at 121°C for 20 minutes. The flasks were cooled to room temperature and inoculated with the above mentioned agar slant. The incubation was carried out for five days on a rotary shaker at 180 rpm and 28°C. 1.5 ml of this culture were mixed with 1.5 ml glycerol (99 %) and stored at -20°C.

EXAMPLE 2

Fermentation of the strain Strepotomycetes sp., DSM 13309 in shake flasks

| Composition of seed medium: | |
|---|---|
| Glucose : | 20 g |
| Soyabean meal : | 10 g |
| CaCO3 : | 0.02 g |
| $CoCl_2$ x 6 $H_2O$ : | 0.001 g |
| Demineralised water: | 1 litre |
| pH : | 6.8-7.0 |

The above medium was distributed in 100 ml amounts in 500 ml Erlenmeyer flasks and autoclaved for 20 mins. The flasks were cooled to room temperature and each flask was inoculated with a loopful of the above mentioned well grown culture of Example 1 and shaken on a rotary shaker for 72 hours at 240 rpm at 27°C (±1°C) to give seed culture.

| Composition of production medium: | |
|---|---|
| Glucose : | 20 g |
| Soyabean meal : | 10 g |
| $CaCO_3$ : | 0.02 g |
| $CoCl_2$ x 6 $H_2O$ : | 0.001 g |
| Demineralised water: | 1 litre |
| pH : | 6.8 - 7.0 |

The production medium was distributed in 100 ml amounts in 500 ml Erlenmeyer flasks and autoclaved at 121°C for 20 min. The flasks were cooled to room temperature and inoculated with the above mentioned seed culture (2% v/v).The fermentation was carried out on a rotary shaker at 240 rpm and 27°C (±1°C) for 72 hours. The production of the inhibitors Citrullimycine A-C was determined by testing their bioactivity.

EXAMPLE 3

Isolation and purification of the Citrullimycines A-C

The culture broth (3 litres) was harvested and freeze dried. The lyophilization product was extracted with methanol (3 litres) and the active extracts were pooled and concentrated under reduced pressure and freeze dried to yield 25 g of crude material. This crude material was purified by preparative HPLC using the following conditions:
1.) Column: MCI® Gel CHP-20P (600 x 40 mm; Kronlab)

| | | | |
|---|---|---|---|
| Eluent: | A) $H_2O$ | | B) MeOH |
| Gradient: | min | %A | %B |
| | 0 | 100 | 0 |
| | 17.5 | 100 | 0 |
| | 17.6 | 80 | 20 |
| | 40 | 80 | 20 |
| | 40.1 | 50 | 50 |
| | 55 | 50 | 50 |
| | 55.1 | 30 | 70 |
| | 67.5 | 30 | 70 |
| | 67.6 | 15 | 85 |
| | 72.5 | 15 | 85 |
| | 72.6 | 10 | 90 |
| | 77.5 | 10 | 90 |
| | 77.6 | 0 | 100 |

Flow: 20 ml/min
Detection : 358 nm

The active fractions eluted after 75 min. The pooled fractions were concentrated under reduced pressure and freeze dried.

Final purification was done by preparative HPLC using the following conditions:
1.) Column: Nucleosil 100-RP 18-AB (5 μ, 250 x 21 mm, Macherey & Nagel)

| | | | |
|---|---|---|---|
| Eluent: | A) $H_2O$ | | B) $CH_3CN$ |
| Gradient: | min | %A | %B |
| | 0 | 70 | 30 |
| | 22 | 70 | 30 |
| | 38 | 52 | 48 |

(continued)

| | | | |
|---|---|---|---|
| Eluent: | A) $H_2O$ | | B) $CH_3CN$ |
| Gradient: | min | %A | %B |
| | 44 | 52 | 48 |
| | 92 | 0 | 100 |
| | 110 | 0 | 100 |
| Flow Rate: 10 ml/min | | | |
| Detection: 360 nm | | | |

The active fractions were analyzed by LC-MS. The Citrullimycines A-C containing fractions eluted after 87 min (A), 97 min (B) and 98 min (C). The pooled fractions were concentrated under reduced pressure and freeze dried. The overall yield from 3 L culture broth was 1 mg of each substance.

The physico chemical and spectral properties of Citrullimycine A-C are given in Tables 1 and 2.

TABLE 1

| | | | |
|---|---|---|---|
| Appearance | : colorless solids | | |
| Solubility | : Methanol, DMSO | | |
| LC-MS (Liquid Chromatography Mass Spectrometry) | : Column: | Purospher STAR RP.18e (Merck, 30 x 2 mm, 3 μm) | |
| | Eluent: | $CH_3CN$/ 10mM $NH_4Ac$ (pH 4.5) | |
| | Gradient: | time % $CH_3CN$ | |
| | | 0.00 | 5.0 |
| | | 6.00 | 100.0 |
| | | 6.50 | 100.0 |
| | | 7.50 | 5.0 |
| | | 8.00 | 5.0 |
| | | 9.00 | 100.0 |
| | | 9.50 | 100.0 |
| | | 10.50 | 5.0 |
| | | 13.00 | 5.0 |
| | Flow: 0.25 ml/min | | |
| | Temp.: 40 °C | | |
| | Detection: 210 nm, 230, 250, 320, 400 (UV); 100-2000 amu (MS) | | |
| Citrullimycine A: | | | |
| Retention time | : 7.3 min | | |
| ESI-MS (Electrospray Ionisation Mass Spectrometry) | : 784.7 amu $(M-H)^-$ | | |
| HR-FAB-MS (High Resolution Fast Atom Bombardment Mass Spectrometry) | : 786.546536 [Calcd for $C_{41}H_{76}N_3O_{11}$: 786.547986 $(M+H)^+$] | | |
| Molecular formula: | $C_{41}H_{75}N_3O_{11}$ | | |
| $MS^n$-Experiments: | Instrument: Finngan LCQ | | |
| | Syringe infusion of sample at 5μL/min | | |

$ESI^+$:
$MS^2$: 786 amu $(M+H^+)$ gives 769 amu $(-NH_3)$,
$MS^3$: 769 amu gives 751 amu $(-H_2O)$
$ESI^-$:
$MS^2$: 784 amu $(M-H)^-$ gives 624 amu $(-C_8H_{16}O_3)$, 610 amu $(-C_9H_{18}O_3)$, 468 amu $(-C_{17}H_{32}O_5)$, 454 amu $(-C_{18}H_{34}O_5)$,
The products at 624 amu and 610 amu are formed in a 25 to 75 ratio.
$MS^3$: 624 amu gives 468 amu $(-C_9H_{16}O_2)$

(continued)

MS$^3$: 610 amu gives 468 amu (-$C_8H_{14}O_2$), 454 amu (-$C_9H_{16}O_2$). The products are formed in a 33 to 66 ratio.
MS$^3$: 468 amu gives 425 amu (-HNCO), 330 amu (-$C_9H_{14}O$), 312 amu (-$C_9H_{16}O_2$), 269 amu (-$C_{10}H_{17}NO_3$)
MS$^4$: 425 amu gives 287 amu (-$C_9H_{14}O$), 269 amu (-$C_9H_{16}O_2$)
MS$^4$: 312 amu gives 269 amu (-HNCO)
MS$^5$: 269 amu gives 225 amu (-$CO_2$), 131 amu (-$C_9H_{14}O$)
MS$^3$: 454 amu gives 411 amu (-HNCO), 330 amu (-$C_8H_{12}O$), 316 amu (-$C_9H_{14}O$), 312 amu ($C_8H_{14}O_2$), 298 amu (-$C_9H_{16}O_2$)
MS$^4$: 411 amu gives 287 amu (-$C_8H_{12}O$), 273 amu (-$C_9H_{14}O$), 269 amu (-$C_8H_{14}O_2$), 255 amu (-$C_9H_{16}O_2$)

• Only nominal masses are given. All given formulas for neutral losses are based on interpretation and are not verified with HR-MS.

| | |
|---|---|
| $^1$H NMR: | see Table 2 |
| $^{13}$C NMR: | see Table 2 |

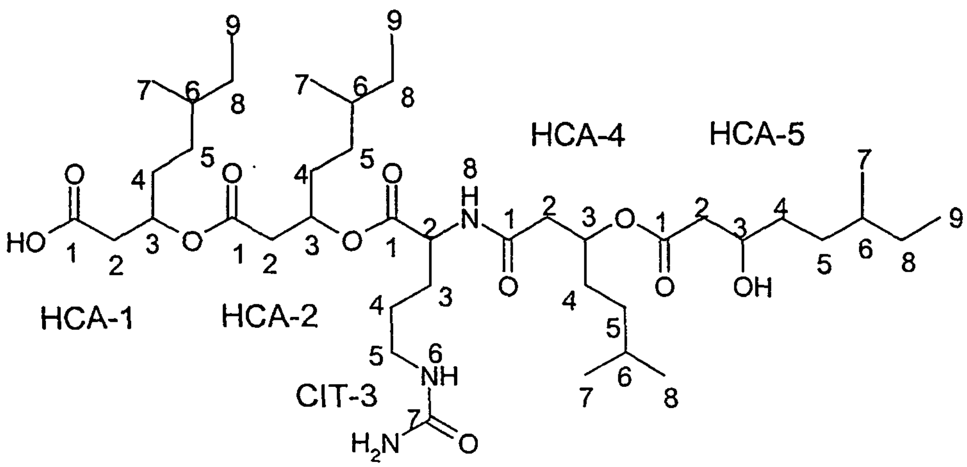

Structure of isomer 1 of Citrullimycine A

| | |
|---|---|
| Citrullimycine B: | |
| Retention time : | 7.1 min |
| Molecular formula : | $C_{40}H_{73}N_3O_{11}$ |
| ESI-MS (Electrospray : Ionisation Mass Spectrometry) | 770 amu (M-H)$^-$ |

MS$^n$ Experiments

ESI$^-$:

$MS^2$: 770 amu $(M-H)^-$ gives 610 amu ($-C_8H_{16}O_3$), 596 amu ($-C_9H_{18}O_3$), 468 amu, 454 amu, 440 amu.
The products at 610 amu and 596 amu are formed in a 1 : 1 ratio, the products at 468 amu, 454 amu and 440 amu are formed in a ratio of 15 : 58 : 27.
$MS^3$: 610 amu gives 468 amu ($-C_8H_{14}O_2$), 454 amu ($-C_9H_{16}O_2$), 440 amu ($-C_{10}H_{18}O_2$). the products are formed in a ratio of 21 : 62 : 17.
$MS^3$: 596 amu gives 468 amu ($-C_7H_{12}O_2$), 454 amu ($-C_8H_{14}O_2$), 440 amu ($-C_9H_{16}O_2$). Ratio: 6 : 51 : 43.
$MS^3$: 468 amu gives 425 amu (-HNCO), 330 amu ($-C_9H_{14}O$), 312 amu ($-C_9H_{16}O_2$), 298 amu ($-C_{10}H_{18}O_2$)
$MS^3$: 454 amu gives 411 amu (-HNCO), 330 amu ($-C_8H_{12}O$), 316 amu ($-C_9H_{14}O$), 312 amu ($C_8H_{14}O_2$), 298 amu ($-C_9H_{16}O_2$)
$MS^3$: 440 amu gives 397 amu (-HNCO), 316 amu ($-C_8H_{12}O$), 298 amu ($-C_8H_{14}O_2$), 284 amu ($-C_9H_{16}O_2$)

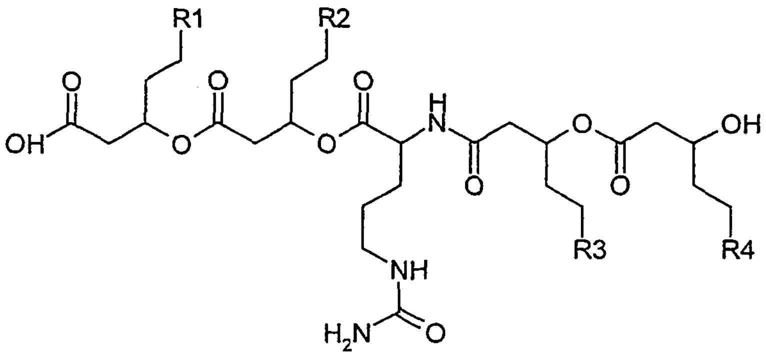

R1-R4 = 2•Bu, 2• Pr
R1-R4 = 1•Bu, 1•Pent, 1•Et, 1• Pr

Structure of Citrullimycin B (mixture of isomers with MW=771 amu)

| | |
|---|---|
| Citrullimycine C: | |
| Retention time : | 7.4 min |
| Molecular formula : | $C_{42}H_{77}N_3O_{11}$ |
| ESI-MS (Electrospray : Ionisation Mass Spectrometry) | 798 amu $(M-H)^-$ |

$MS^n$ Experiments

$ESI^-$:

$MS^2$: 798 amu $(M-H)^-$ gives 638 amu ($-C_8H_{16}O_3$), 624 amu ($-C_9H_{18}O_3$), 610 amu ($-C_{10}H_{20}O_3$) The products are formed in the ratio of 5 : 90 : 5.
$MS^3$: 624 amu gives 482 amu ($-C_8H_{14}O_2$), 468 amu ($-C_9H_{16}O_2$), 454 amu ($-C_{10}H_{18}O_2$), 440 amu ($-C_{11}H_{20}O_2$). Ratio: 1.6 : 96 : 2 : 0.4.
$MS^4$: 468 amu gives 425 amu (-HNCO), 330 amu ($-C_9H_{14}O$), 312 amu ($-C_9H_{16}O_2$), 287 amu ($-C_{10}H_{15}O_2N$), 269 amu ($-C_{10}H_{17}O_3N$)
$MS^5$: 425 amu gives 287 amu ($-C_9H_{14}O$), 269 amu ($-C_9H_{16}O_2$)
$MS^5$: 312 amu gives 269 amu (-HNCO)
$MS^5$: 269 amu gives 225 amu ($-CO_2$), 131 amu ($-C_9H_{14}O$)

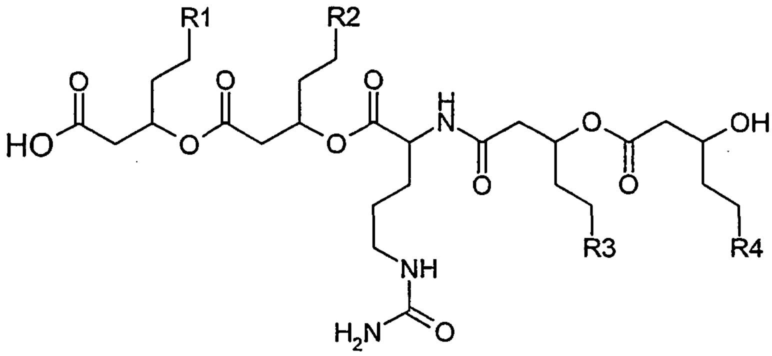

R1-R4 = 4•Bu
R1-R3 = 2•Bu, 1•Pent, R4 = Pr
R1-R3 = 2•Bu, 1•Pr, R4 = Pent

Structure of Citrullimycin C (mixture of isomers, MW=799 amu)

Table 2:

| $^1$H and $^{13}$C NMR Spectroscopic Data of Citrullimycine A in MeOD at 300 K[a]. | | |
|---|---|---|
| | $^1$H | $^{13}$C |
| HCA1-1[a] | - | 171.58[a] |
| 2 | 2.59 | 40.30 |
| 3 | 5.18 | 72.21 |
| 4 | 1.62 | 32.90 |
| 5 | b) | b) |
| 6 | b) | b) |
| 7 | 0.87 | 19.45 |
| 8 | 1.35/1.15 | 30.51 |
| 9 | 0.87 | 11.73 |
| HCA2-1[a] | - | 171.44[a] |
| 2 | 2.58 | 40.24 |
| 3 | 5.16 | 72.61 |
| 4 | 1.62 | 32.90 |
| 5 | b) | b) |
| 6 | b) | b) |
| 7 | 0.87 | 19.45 |
| 8 | 1.35/1.15 | 30.51 |
| 9 | 0.87 | 11.73 |
| CIT3-1 | - | 175.81 |
| 2 | 4.33 | 53.99 |
| 3 | 1.87/1.68 | 30.25 |
| 4 | 1.55 | 27.73 |

(continued)

| $^1H$ and $^{13}C$ NMR Spectroscopic Data of Citrullimycine A in MeOD at 300 K[a)]. | | |
|---|---|---|
| | $^1H$ | $^{13}C$ |
| 5 | 3.12 | ~40.6 (a) |
| 6 | - | - |
| 7 | - | 162.21 |
| HCA4-1 | - | 172.31 |
| 2 | 2.55/2.48 | 41.51 |
| 3 | 5.19 | 73.35 |
| 4 | 1.63 | 32.54 |
| 5 | 1.22 | 35.45 |
| 6 | 1.54 | 29.05 |
| 7 | 0.89 | 23.00 |
| 8 | 0.89 | 23.00 |
| HCA5-1 | - | 172.85 |
| 2 | 2.44 | 43.74 |
| 3 | 3.94 | 69.65 |
| 4 | 1.47 | 35.69 |
| 5 | 1.36 | 33.40 |
| 6 | b) | b) |
| 7 | 0.87 | 19.45 |
| 8 | 1.35/1.15 | 30.51 |
| 9 | 0.87 | 11.73 |
| a) The data of HCA1 und HCA2 are interchangeable.<br>b) No assignment possible. | | |

Example 4:

Bioactivity Assay

SPA [3H] Neurotensin Receptor binding Assay:

Compounds with an affinity for the neurotensin receptor will displace the binding of [3H] neurotensin which results in a diminished radioactive signal. For the SPA method, receptors which are immobilized directly to PVT WGA (Wheat Germ Agglutinin) coated SPA beads (Amersham pharmacia) should bind the radiolabelled ligand. The ligand-receptor complex will be held in close enough proximity to stimulate the beads to emit light. Any unbound radioligand is too distant from the bead to transfer energy and therefore will not be detected. The samples were pre-diluted 1:5 with assay buffer (50 mM Tris-HCl buffer with 1 mM EDTA, 0.2 mM Bacitracin and 0.1 % BSA, pH 7.4) in deep well plates. The final dilution in the assay was 1:20.

96-well isoplates from Waffac were used for the screening. Each well received: 50 μl of sample, 50 μl of membrane in assay buffer (final conc. 16μg/well), 50 μl of PVT-VGA beads (final conc. 0.75 μg/well) and 50μl of 4 nM [$^3$H]neurotensin. The plates were sealed and incubated for 2 hours on a shaker (1100 rpm) at room temperature. Prior to counting (MicroBeta Trilux, Wallac) the beads were allowed to settle for at least 20 minutes.

On each plate four wells without samples were used to determine the total receptor ligand binding and another four wells with 1 μM (L-α, γ-diaminobutyryl) neurotensin were used to determine the nonspecific binding respectively. Inhibition activities are expressed as:

[1-(dpm sample–dpm nonspecific) / (dpm total binding–dpm nonspec.)] x 100 (%)

The activity of the Citrullimycines is in the range of 16-30μM. The $IC_{50}$ of Citrullimycine A was determined as 16μM.

## Claims

1. A compound of the formula I:

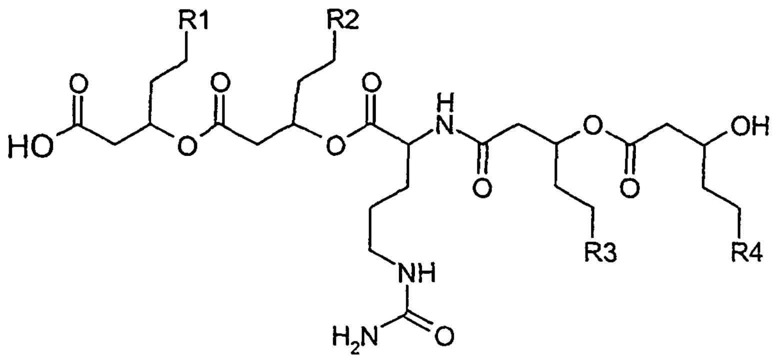

where
$R_1$, $R_2$, $R_3$ and $R_4$ are, independently of one another, alkyl residues with 1 to 6 carbon atoms; and the physiologically tolerated salts and derivatives thereof, in all their stereoisomeric forms and all their tautomeric forms.

2. A compound of the formula I as claimed in claim 1 or claim 2, in which one, two, three or all of $R_1$ to $R_4$ are butyl residues, and the physiologically tolerated salts and derivatives thereof, in all their stereoisomeric forms and all their tautomeric forms.

3. A compound of the formula I as claimed in any one of the preceding claims in which $R_1$ to $R_4$ consist of three butyl residues and one propyl residue, and the physiologically tolerated salts and derivatives thereof , in all their stereoisomeric forms and all their tautomeric forms.

4. A compound of the formula I as claimed in any one of claims 1 to 3, in which $R_1$ to $R_4$ comprise two butyl and two propyl residues or one butyl, one pentyl, one ethyl and one propyl residue, and the physiologically tolerated salts and derivatives thereof, in all their stereoisomeric forms and all their tautomeric forms.

5. A compound of the formula I as claimed in any one of claims 1 to 3, in which $R_1$ o $R_4$ comprise four butyl residues or two butyl, one propyl and one pentyl residue, and the physiologically tolerated salts and derivatives thereof , in all their stereoisomeric forms and all their tautomeric forms.

6. A compound of the formula I as claimed in any one of the preceding claims comprising a mixture of two or more isomers.

7. A compound of the formula 1 or a physiologically tolerated salt or derivative thereof as claimed in one or more of claims 1 to 6, obtainable by cultivating Streptomycete sp., DSM 13309 or one of its variants or mutants under aerobic conditions in a nutrient medium containing sources of carbon and nitrogen, isolating and purifying one or more target compounds in a customary manner, and converting where appropriate into physiologically tolerated salts or derivatives, in all their stereoisomeric and tautomeric forms.

8. A process for the production of a compound of the formula I or a salt or equivalent thereof as claimed in one or more

of claims 1 to 6, comprising cultivating streptomycete sp., DSM 13309 or one of its variants or mutants under aerobic conditions in a nutrient medium containing sources of carbon and nitrogen, isolating and purifying one or more target compounds in a customary manner, and converting where appropriate into physiologically tolerated salts or derivatives, in all their stereoisomeric and tautomeric forms.

9. The process as claimed in claim 8, wherein the cultivation is carried out at a temperature between 20 - 35°C and a pH between 5 and 8.

10. A compound of the formula I or a physiologically tolerated salt or derivative thereof as claimed in one or more of claim 1 to 6 for use as a pharmaceutical.

11. A compound of the formula I or a physiologically tolerated salt or derivative thereof as claimed in one or more of claim 1 to 6 for use as an inhibitor of neurotensin receptor.

12. The use of a compound of the formula I or a physiologically tolerated salt thereof as claimed in one or more of claims 1 to 6 for the manufacture of a pharmaceutical for the treatment of schizophrenia , Parkinson's or Alzheimer's disease.

13. A pharmaceutical composition, comprising an effective amount of a compound of the formula I as claimed in one or more of claims 1 to 6, or a pharmaceutically acceptable salt or derivative thereof , and a pharmaceutically acceptable carrier.

14. A method for the production of a pharmaceutical as claimed in claim 13, which comprises converting at least one compound of the formula I or a physiologically tolerated salt thereof as claimed in one or more of claims 1 to 6 with suitable excipients and/or carriers into a suitable dosage form.

15. Streptomycetes species, DSM 13309.

**Patentansprüche**

1. Verbindung der Formel I:

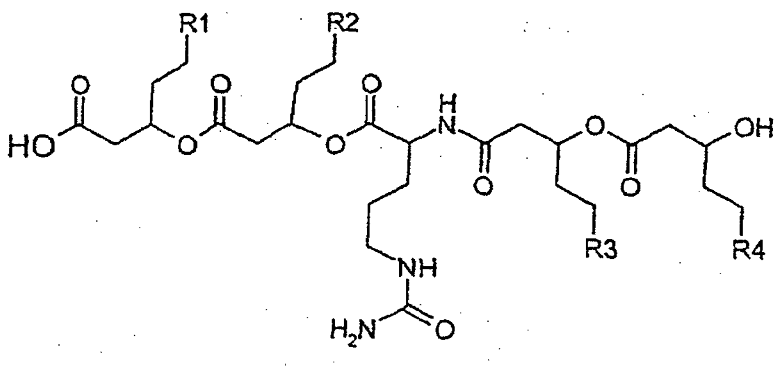

worin
$R_1$, $R_2$, $R_3$ und $R_4$ unabhängig voneinander jeweils für Alkylreste mit 1 bis 6 Kohlenstoffatomen stehen, sowie deren physiologisch unbedenkliche Salze und Derivate, mit allen ihren stereoisomeren Formen und allen ihren tautomeren Formen.

2. Verbindung der Formel I nach Anspruch 1, bei der einer, zwei, drei oder alle der Reste $R_1$ bis $R_4$ Butylreste sind, sowie deren physiologisch unbedenkliche Salze und Derivate, mit allen ihren stereoisomeren Formen und allen ihren tautomeren Formen.

3. Verbindung der Formel I nach einem der vorhergehenden Ansprüche, bei der $R_1$ bis $R_4$ aus drei Butylresten und einem Propylrest bestehen, sowie deren physiologisch unbedenkliche Salze und Derivate, mit allen ihren stereoisomeren Formen und allen ihren tautomeren Formen.

**4.** Verbindung der Formel 1 nach einem der Ansprüche 1 bis 3, bei der $R_1$ bis $R_4$ zwei Butyl- und zwei Propylreste oder einen Butyl-, einen Pentyl-, einen Ethyl- und einen Propylrest umfassen, sowie deren physiologisch unbedenkliche Salze und Derivate, mit allen ihren stereoisomeren Formen und allen ihren tautomeren Formen.

**5.** Verbindung der Formel 1 nach einem der Ansprüche 1 bis 3, bei der $R_1$ bis $R_4$ vier Butylreste oder zwei Butyl-, einen Propyl- und einen Pentylrest umfassen, sowie deren physiologisch unbedenkliche Salze und Derivate, mit allen ihren stereoisomeren Formen und allen ihren tautomeren Formen.

**6.** Verbindung der Formel I nach einem der vorhergehenden Ansprüche, umfassend ein Gemisch aus mindestens zwei Isomeren.

**7.** Verbindung der Formel I oder physiologisch unbedenkliches Salz oder Derivat davon nach einem oder mehreren der Ansprüche 1 bis 6, erhältlich durch Kultivieren von Streptomyces sp. DSM 13309 oder einer seiner Varianten oder Mutanten unter aeroben Bedingungen in einem Kohlenstoff- und Stickstoffquellen enthaltenden Nährmedium, Isolieren und Aufreinigen einer oder mehrerer Zielverbindungen auf übliche Weise und gegebenenfalls Umwandeln in physiologisch unbedenkliche Salze oder Derivate, mit allen ihren stereoisomeren und tautomeren Formen.

**8.** Verfahren zur Herstellung einer Verbindung der Formel I oder eines Salzes oder Äquivalents davon nach einem oder mehreren der Ansprüche 1 bis 6, umfassend das Kultivieren von Streptomyces sp. DSM 13309 oder einer seiner Varianten oder Mutanten unter aeroben Bedingungen in einem Kohlenstoff- und Stickstoffquellen enthaltenden Nährmedium, Isolieren und Aufreinigen einer oder mehrerer Zielverbindungen auf übliche Weise und gegebenenfalls Umwandeln in physiologisch unbedenkliche Salze oder Derivate, mit allen ihren stereoisomeren und tautomeren Formen.

**9.** Verfahren nach Anspruch 8, wobei die Kultivierung bei einer Temperatur zwischen 20 - 35 °C und einem pH-Wert zwischen 5 und 8 durchgeführt wird.

**10.** Verbindung der Formel I oder physiologisch unbedenkliches Salz oder Derivat davon nach einem oder mehreren der Ansprüche 1 bis 6 zur Verwendung als Arzneimittel.

**11.** Verbindung der Formel I oder physiologisch unbedenkliches Salz oder Derivat davon nach einem oder mehreren der Ansprüche 1 bis 6 zur Verwendung als Inhibitor des Neurotensin-Rezeptors.

**12.** Verwendung einer Verbindung der Formel I oder eines physiologisch unbedenklichen Salzes oder Derivats davon nach einem oder mehreren der Ansprüche 1 bis 6 zur Herstellung eines Arzneimittels zur Behandlung von Schizophrenie, Morbus Parkinson oder Morbus Alzheimer.

**13.** Pharmazeutische Zusammensetzung, umfassend eine wirksame Menge einer Verbindung der Formel I nach einem oder mehreren der Ansprüche 1 bis 6 oder eines pharmazeutisch unbedenklichen Salzes oder Derivats davon sowie einen pharmazeutisch unbedenklichen Träger.

**14.** Verfahren zur Herstellung eines Arzneimittels nach Anspruch 13, bei dem man wenigstens eine Verbindung der Formel I oder ein physiologisch unbedenkliches Salz davon nach einem oder mehreren der Ansprüche 1 bis 6 zusammen mit geeigneten Hilfsstoffen und/oder Trägern in eine geeignete Dosierungsform umwandelt.

**15.** Streptomyces-Spezies DSM 13309.

**Revendications**

**1.** Composé de la formule I :

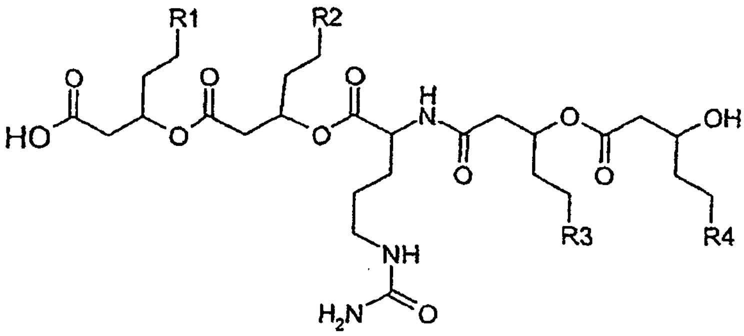

dans laquelle
$R_1$, $R_2$, $R_3$ et $R_4$ représentent, indépendamment les uns des autres, des résidus alkyle avec d'un à six atomes de carbone ; ainsi que les sels et dérivés tolérés physiologiquement de celui-ci, dans toutes leurs formes stéréoisomères et toutes leurs formes tautomères.

**2.** Composé de la formule I selon la revendication 1, dans lequel un, deux, trois des $R_1$ à $R_4$ ou tous les $R_1$ à $R_4$ sont des résidus butyle, et les sels et dérivés physiologiquement tolérés de celui-ci, dans toutes leurs formes stéréoisomères et toutes leurs formes tautomères.

**3.** Composé de la formule I selon l'une quelconque des revendications précédentes, dans lequel $R_1$ à $R_4$ sont constitués de trois résidus butyle et d'un résidu propyle, et les sels et dérivés physiologiquement tolérés de celui-ci, dans toutes leurs formes stéréoisomères et toutes leurs formes tautomères.

**4.** Composé de la formule I selon l'une quelconque des revendications 1 à 3, dans lequel $R_1$ à $R_4$ comprennent deux résidus butyle et deux résidus propyle ou un résidu butyle, un résidu pentyle, un résidu éthyle et un résidu propyle, ainsi que les sels et dérivés physiologiquement tolérés de celui-ci, dans toutes leurs formes stéréoisomères et toutes leurs formes tautomères.

**5.** Composé de la formule I selon l'une quelconque des revendications 1 à 3, dans lequel $R_1$ à $R_4$ comprennent quatre résidus butyle ou deux résidus butyle, un résidu propyle et un résidu pentyle, ainsi que les sels et dérivés physiologiquement tolérés de celui-ci, dans toutes leurs formes stéréoisomères et toutes leurs formes tautomères.

**6.** Composé de la formule I selon l'une quelconque des revendications précédentes qui comprend un mélange de deux isomères ou plus.

**7.** Composé de la formule I ou un sel ou un dérivé physiologiquement toléré de celui-ci selon une ou plusieurs des revendications 1 à 6, lesquels peuvent être obtenus par la culture de Sterptomycete sp., (DSM 13309), ou d'une de ses variantes ou d'un de ses mutants dans des conditions aérobies dans un milieu nutritif qui comprend des sources de carbone et d'azote, l'isolation et la purification d'un ou de plusieurs des composés cibles d'une manière habituelle, et leur conversion s'il y a lieu en des sels ou dérivés physiologiquement tolérés, dans toutes leurs formes stéréoisomères et tautomères.

**8.** Procédé pour la production d'un composé de la formule I ou d'un sel ou d'un dérivé physiologiquement toléré de celui-ci selon une ou plusieurs des revendications 1 à 6, lequel procédé comprend la culture de Sterptomycete sp., (DSM 13309), ou d'une de ses variantes ou d'un de ses mutants dans des conditions aérobies dans un milieu nutritif qui comprend des sources de carbone et d'azote, l'isolation et la purification d'un ou de plusieurs des composés cibles d'une manière habituelle, et leur conversion s'il y a lieu en des sels ou dérivés physiologiquement tolérés, dans toutes leurs formes stéréoisomères et tautomères.

**9.** Procédé selon la revendication 8, dans lequel la culture est effectuée à une température située entre 20 et 35 °C et à un pH situé entre 5 et 8.

**10.** Composé de la formule I ou un sel ou un dérivé physiologiquement toléré de celui-ci selon une ou plusieurs des revendications 1 à 6, pour utilisation comme médicament.

**11.** Composé de la formule I ou un sel ou un dérivé physiologiquement toléré de celui-ci selon une ou plusieurs des revendications 1 à 6, pour utilisation comme inhibiteur du récepteur de la neurotensine.

**12.** Composé de la formule I ou un sel ou un dérivé physiologiquement toléré de celui-ci selon une ou plusieurs des revendications 1 à 6, pour la fabrication d'un médicament utilisé dans le traitement de la schizophrénie, de la maladie de Parkinson ou de la maladie d'Alzheimer.

**13.** Composition pharmaceutique qui comprend une quantité efficace d'un composé de la formule I selon une ou plusieurs des revendications 1 à 6, ou d'un sel ou d'un dérivé pharmaceutiquement acceptable de celui-ci, et d'un vecteur pharmaceutiquement acceptable.

**14.** Procédé de fabrication d'un médicament selon la revendication 13, qui comprend la conversion d'au moins un composé de la formule I ou d'un sel physiologiquement toléré de celui-ci selon une ou plusieurs des revendications 1 à 6 avec des excipients et/ou des vecteurs dans une forme pharmaceutique appropriée.

**15.** Espèces DSM 13309 de Streptomycetes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Advanced Organic Synthesis. John Wiley & Sons, 1992, 395
- Advanced Organic Synthesis. Wiley & Sons, 1992, 1213